# EUROPEAN PATENT APPLICATION

(11) **EP 4 443 346 A1**
(43) Date of publication of application: **09.10.2024**
(21) Application number: 22901384.2
(22) Date of filing: 30.11.2022
(51) Int. Cl.: G06N 20/00, A61B 6/03, G06T 7/00

(54) **ESTIMATION DEVICE, ESTIMATION METHOD, AND PROGRAM**

(30) Priority: 01.12.2021 JP 2021195421
(71) Applicant: Tokyo Institute of Technology, Tokyo 152-8550 (JP)
(72) Inventor: SUZUKI Kenji, Tokyo 152-8550 (JP)
(74) Representative: Oak & Fox
(86) International application number: PCT/JP2022/044260
(87) International publication number: WO 2023/100953

(57) **Abstract**

The present invention makes it possible to analyze the reason for estimation when estimation is implemented by a model constructed through machine learning. A data acquisition unit (11) acquires input data (IN). An estimation processing unit (12) estimates information relating to an estimation object represented by a signal included in the input data (IN) by inputting the input data (IN) into a trained model (100). An image display unit (13) displays the information relating to the estimation object and the reason for estimation. The trained model (100) includes a plurality of modularized networks constructed in such a manner that characteristics of the estimation object represented by a signal included in first training image data are divided by type and training is implemented in advance, and a fusion network constructed in such a manner that a plurality of output signals obtained by input of second training data into the plurality of modularized networks are input and supervised training is implemented, whereby estimating information relating to the estimation object represented by a signal included in an input image, according to a signal to be input.

## Description

### Technical Field

The present invention relates to an estimation apparatus, an estimation method, and a program that can present determination reason and basis, for example.

### Background Art

In recent years, the use of machine learning called deep learning is progressing in various fields, for example, robots, medicine, image understanding, vehicles, and voice recognition. For example, in the medical field, the machine learning is applied to an image interpretation support for medical images that forms images of a state inside a living body.

As an imaging method for medical images, for example, a CT (Computed Tomography) apparatus is known. The CT apparatus can image an X-ray absorption state of a human body, and form images of tissues and morphology inside the human body. As another imaging method, a magnetic resonance imaging (MRI) apparatus is known. The MRI apparatus acquires two-dimensional or three-dimensional image information, for example, by applying a magnetic field to a tissue of the human body and using a resulting nuclear magnetic resonance (NMR) phenomenon. The MRI apparatus has excellent features, such as the capability of imaging tissues that cannot be imaged by a computed tomography (CT) apparatus and the absence of radiation exposure. For these apparatuses, a method has also been proposed to automatically perform diagnosis based on imaged data (see Patent Literature 1).

Attempts are being made to input images imaged by such a CT apparatus or MRI apparatus into a model constructed in advance through training of the machine-learning model to estimate lesions included in the images.

### Citation List

### Patent Literature

[Patent Literature 1]
Japanese Translation of PCT International Application Publication No. 2020-524018

### Summary of Invention

### Technical Problem

However, the general machine learning method such as deep learning described above is a paradigm in end-to-end machine learning in which training is performed in a single model from input data to the final result all at once. For this reason, although an estimation result can be obtained as a result of input of unknown input data into a model, it is not possible to know the reason for determination or the basis of how the estimation result was obtained. In other words, the general machine learning method such as deep learning has properties of a "black box". Further, since a model network constructed by the deep learning has a deep and complex structure, it is difficult in principle to theoretically analyze internal representation of the network or analyze an internal state.

Defects of such deep learning can become obstacles to commercialization and social implementation of applied products for deep learning. In other words, when social implementation is performed by the deep learning without being aware of what kind of learning is being accomplished, the deep learning will behave in unexpected ways, leading to serious problems in applications in life-related fields such as medicine and transportation.

The present invention has been made in view of the above circumstances, and an object thereof is to make it possible to analyze reasons for estimation when estimation is performed using a model constructed by machine learning.

### Solution to Problem

An aspect of the present invention is an estimation apparatus including: a data acquisition unit configured to acquire input data serving as image data; an estimation processing unit configured to estimate information relating to an estimation object represented by a signal included in the input data by inputting the input data into a trained model; and a display unit configured to display the estimated information relating to the estimation object and information indicating a reason why the estimated information relating to the estimation object is estimated, in which the trained model includes a plurality of modularized networks constructed in such a manner that characteristics of the estimation object represented by a signal included in first training image data are divided by type and training is implemented in advance, and a fusion network constructed in such a manner that a plurality of output signals obtained by input of second training image data into the plurality of modularized networks are input and supervised training is implemented, whereby estimating information relating to the estimation object represented by a signal included in an input image serving as image data, according to a signal to be input.

An aspect of the present invention is the estimation apparatus described above, in which the information relating to the estimation object includes some or all of name, type, and attribute of an object and a numerical value related to the object.

An aspect of the present invention is the estimation apparatus described above, in which the fusion network is constructed by training of a multidimensional vector having the plurality of output signals as components.

An aspect of the present invention is the estimation apparatus described above, in which the estimation processing unit further outputs information indicating the degree of influence of the plurality of output signals on a result of estimation.

An aspect of the present invention is the estimation apparatus described above, in which the estimation processing unit selects the predetermined number of the output signals in descending order of the degree of influence on the result of estimation, and further outputs information indicating the selected output signals.

An aspect of the present invention is the estimation apparatus described above, in which the display unit displays the information indicating the selected output signals as information indicating the reason why the information relating to the estimation object is obtained or information indicating the reason and a basis on which the reason is obtained.

An aspect of the present invention is the estimation apparatus described above, in which the estimation processing unit is configured to construct the plurality of trained modularized networks by receiving the first training image data from the data acquisition unit and inputting the first training image data into the plurality of modularized networks, and construct the trained fusion network by inputting the plurality of output signals into the fusion network to implement supervised training.

An aspect of the present invention is the estimation apparatus described above, in which the first training image data includes a plurality of data sets trained in each of the plurality of modularized networks, the plurality of data sets corresponding to the information relating to the estimation object, and the plurality of modularized networks, which have been trained, are constructed in such a manner that the plurality of data sets are input into the plurality of modularized networks to be subjected to training, respectively.

An aspect of the present invention is the estimation apparatus described above, in which each of the plurality of output signals obtained by inputting the second training image data into the plurality of modularized networks is a signal corresponding to one type of the characteristics of the estimation object.

An aspect of the present invention is the estimation apparatus described above, in which the estimation processing unit estimates, as the information relating to the estimation object, discrimination information relating to a state of the estimation object discriminated based on a type of the characteristics of the estimation object.

An aspect of the present invention is the estimation apparatus described above, in which the fusion network constructed by training of the first training image data and the second training image data includes a boundary surface that is formed in a vector space, to which a multidimensional vector having the output signals from the plurality of modularized networks belongs, to discriminate the state of the estimation object.

An aspect of the present invention is an estimation method including: acquiring input data serving as image data; estimating information relating to an estimation object represented by a signal included in the input data by inputting the input data into a trained model; and displaying the estimated information relating to the estimation object and information indicating a reason why the estimated information relating to the estimation object is estimated, in which the trained model includes a plurality of modularized networks constructed in such a manner that characteristics of the estimation object represented by a signal included in first training image data are divided by type and training is implemented in advance, and a fusion network constructed in such a manner that a plurality of output signals obtained by input of second training image data into the plurality of modularized networks are input and supervised training is implemented, whereby estimating information relating to the estimation object represented by a signal included in an input image serving as image data, according to a signal to be input.

An aspect of the present invention is a program causing a computer to execute processes of: acquiring input data serving as image data; estimating information relating to an estimation object represented by a signal included in the input data by inputting the input data into a trained model; and displaying the estimated information relating to the estimation object and information indicating a reason why the estimated information relating to the estimation object is estimated, in which the trained model includes a plurality of modularized networks constructed in such a manner that characteristics of the estimation object represented by a signal included in first training image data are divided by type and training is implemented in advance, and a fusion network constructed in such a manner that a plurality of output signals obtained by input of second training image data into the plurality of modularized networks are input and supervised training is implemented, whereby estimating information relating to the estimation object represented by a signal included in an input image serving as image data, according to a signal to be input.

### Advantageous Effects of Invention

According to the present invention, it is possible to analyze reasons for estimation when estimation is performed using a model constructed by machine learning.

### Brief Description of Drawings

Fig. 1 is a diagram showing an example of a hardware configuration for realizing an estimation apparatus according to a first embodiment;
Fig. 2 is a diagram schematically showing a configuration of the estimation apparatus according to the first embodiment;
Fig. 3 is a flowchart of estimation processing in the estimation apparatus according to the first embodiment;
Fig. 4 is a diagram schematically showing a configuration of a trained model according to the first embodiment;
Fig. 5 is a diagram showing examples of characteristics of nodules corresponding to modularized networks, respectively, and of training images with respect to types of the nodules trained for network construction, respectively;
Fig. 6 is a diagram schematically showing an example of estimation in the estimation apparatus according to the first embodiment;
Fig. 7 is a diagram showing an image of an estimation process in the example of Fig. 5;
Fig. 8 is a diagram showing estimation accuracy in estimation processing using the estimation apparatus and a general model according to the present embodiment;
Fig. 9 is a flowchart of training processing in an estimation apparatus according to a second embodiment;
Fig. 10 is a diagram schematically showing a first example of input/output of data in the estimation apparatus in the second embodiment; and
Fig. 11 is a diagram schematically showing a second example of input/output of data in the estimation apparatus in the second embodiment.

### Description of Embodiments

The specific embodiments will be described with reference to the drawings. However, the present invention is not limited to the following embodiments. In order to clarify the description, the following descriptions and drawings will be simplified as appropriate. The same elements are denoted by the same reference signs, and duplicate descriptions will be omitted.

### First Embodiment

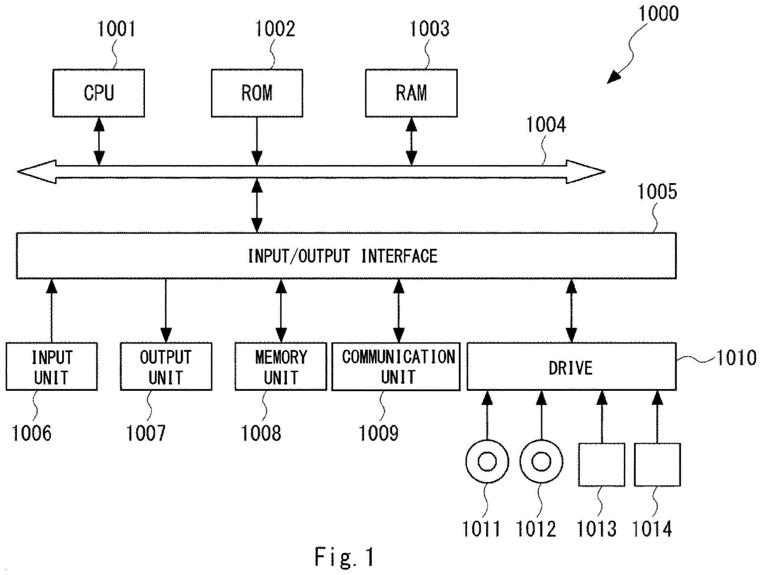

First, as a premise for understanding an estimation apparatus according to a first embodiment, an example of a hardware configuration for realizing an estimation apparatus will be described. Fig. 1 is a diagram showing an example of a hardware configuration for realizing the estimation apparatus according to the first embodiment. An estimation apparatus 10 can be realized by a computer 1000 such as a dedicated computer or a personal computer (PC). However, the computer need not be physically single, and may be multiple when performing distributed processing. As shown in Fig. 1, the computer 1000 includes a central processing unit (CPU) 1001, a read only memory (ROM) 1002 and a random-access memory (RAM) 1003, which are connected to each other via a bus 1004. Although the description of the OS software for operating the computer or the like will be omitted, it is assumed that the computer for constructing the estimation apparatus also naturally has one.

An input/output interface 1005 is also connected to the bus 1004. For example, an input unit 1006 configured of a keyboard, mouse, sensor, or the like, an output unit 1007 configured of a display configured of a CRT, LCD, or the like, and a headphone, speaker, or the like, a memory unit 1008 configured of a hard disk, or the like, and a communication unit 1009 configured of a modem, terminal adapter, or the like, are connected to the input/output interface 1005.

The CPU 1001 performs various types of processing according to various programs stored in the ROM 1002 or loaded into the RAM 1003 from the memory unit 1008, and in the present embodiment, for example, processing of various units of the estimation apparatus 10 described later. Note that, a GPU (Graphics Processing Unit) may be provided to perform various types of processing according to various programs stored in the ROM 1002 or loaded into the RAM 1003 from the memory unit 1008, as the CPU 1001, and in the present embodiment, for example, processing of various units of the estimation apparatus 10 described later. Note that, the GPU is suitable for applications in which routine processing is performed in parallel, and by applying it to processing in neural networks, which will be described later, it is possible to improve the processing speed compared to the CPU 1001. Data necessary for the CPU 1001 and the GPU to perform various types of processing are also stored in the RAM 1003 as appropriate.

For example, the communication unit 1009 performs communication processing via the Internet (not shown), transmits data provided by the CPU 1001, and outputs data received from the communication partner to the CPU 1001, the RAM 1003, and the memory unit 1008. The memory unit 1008 communicates with the CPU 1001 to store and erase information. The communication unit 1009 also performs communication processing of analog or digital signals with other apparatuses.

Further, the input/output interface 1005 is also connected to a drive 1010 as needed, for example, a magnetic disk 1011, an optical disk 1012, a flexible disk 1013, or a semiconductor memory 1014 is appropriately provided to the input/output interface 1005, and a computer program read from them is installed in the memory unit 1008 as needed.

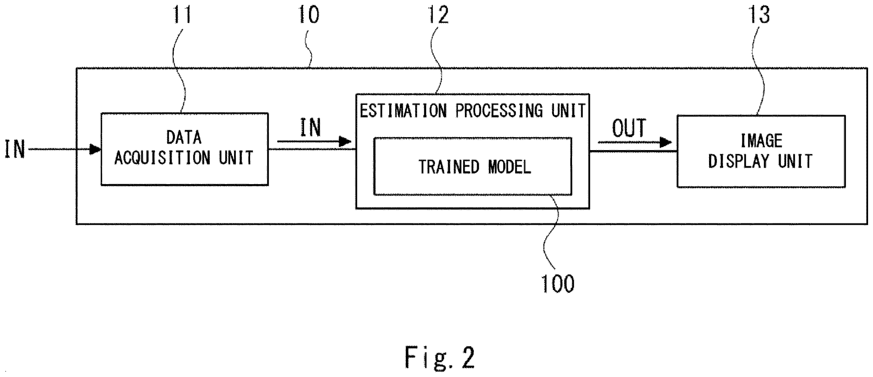

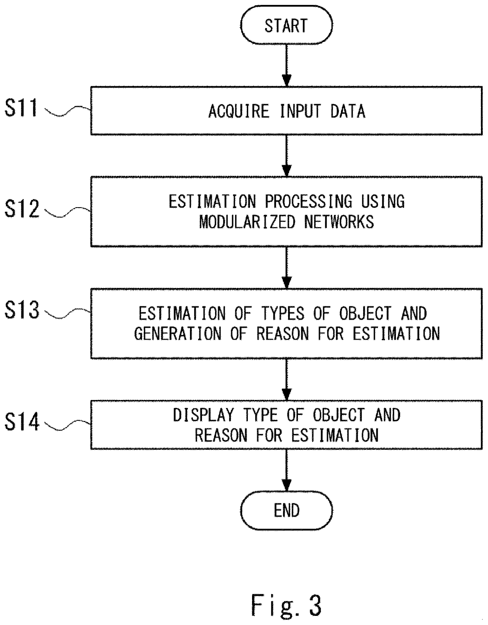

A configuration and operation of the estimation apparatus 10 according to the first embodiment will be described below. Fig. 2 schematically shows a configuration of the estimation apparatus 10 according to the first embodiment. Fig. 3 shows a flowchart of estimation processing in the estimation apparatus 10 according to the first embodiment. The estimation apparatus 10 includes a data acquisition unit 11, an estimation processing unit 12, and an image display unit 13. The image display unit 13 can also display text, sentences, and natural language.

The data acquisition unit 11 reads input data IN that is image data obtained by imaging with a CT apparatus or an MRI apparatus (step S11 in Fig. 3). The read input data IN is output to the estimation processing unit 12. The data acquisition unit 11 can read the input data IN from a memory apparatus or the CT apparatus or MRI apparatus.

The estimation processing unit 12 holds a trained model that has been constructed in advance, and inputs the input data IN to be analyzed into the held trained model, thereby outputting an estimation result OUT estimated based on the training result (steps S12 and S13 in Fig. 3). Note that, the estimation here means that the input data IN is input into the trained model to estimate information about an estimation object represented by a signal included in the input data IN, for example, the name, type, attribute, and related numerical values of the object to be estimated and to generate the reason that leads to the estimation result. The trained model used by the estimation processing unit 12 will be described below.

The estimation processing unit 12 may receive information indicating the trained model from an external memory apparatus or the like and hold the trained model based on the information indicating the received trained model. The information indicating the trained model may be provided in the form of, for example, a parameter specifying a weighting factor between neurons of a neural network constituting the trained model.

Based on the estimation result OUT received from the estimation processing unit 12, the image display unit 13 displays the estimation result on a display apparatus such as a display (step S14 in Fig. 3). For example, the output unit 1007 described above may be used as the image display unit 13.

Next, a trained model and estimation processing according to the present embodiment will be described. A trained model 100 is configured to estimate information about input data, for example, the name, type, attribute, and related numerical values of an object and to generate the reason that leads to the estimation result by performing first-stage estimation using a plurality of modularized networks and further performing second-stage estimation using the estimation result.

In the present embodiment, CT image data of a chest of a subject is used as input data to be estimated. The object to be estimated is a nodule included in the CT image data that is suspected of being a malignant or benign tumor, or the like, the type of the nodule is estimated, and the estimation result is output.

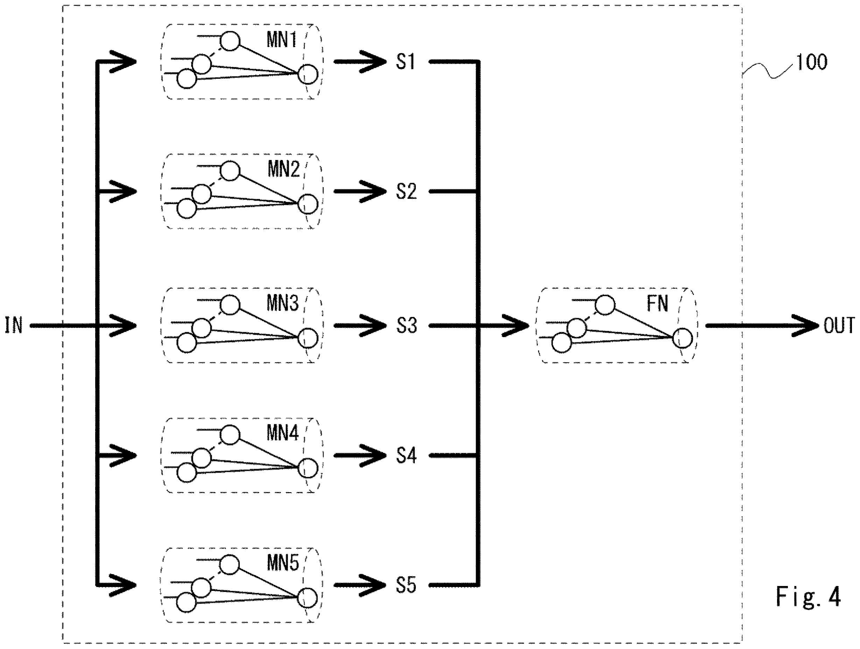

Fig. 4 schematically shows a configuration of the trained model 100. The trained model includes a plurality of modularized networks prepared according to the type of object (a nodule in this example) included in image data that is input data, and a fusion network FN through which the estimation result is output based on the output from the plurality of modularized networks. Here, a case will be described as an example in which the plurality of modularized networks includes five modularized networks MN1 to MN5.

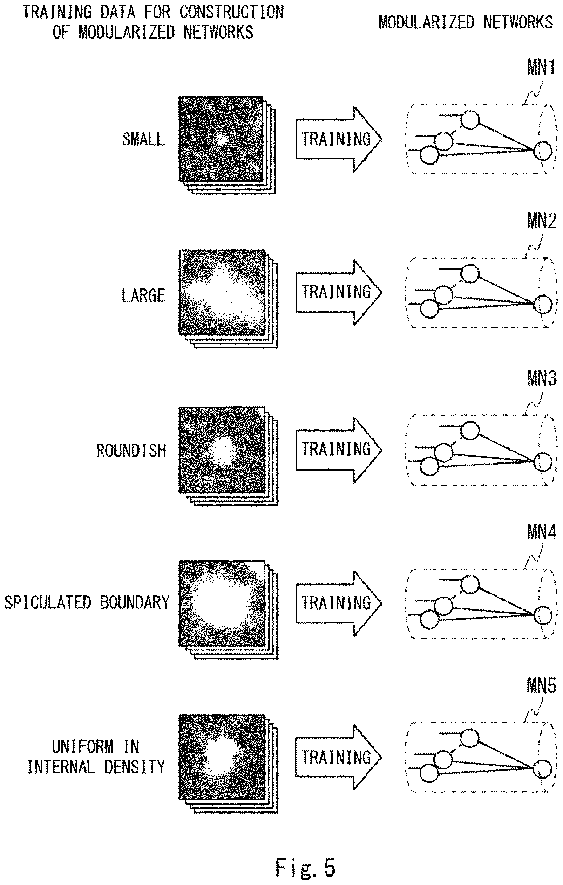

The modularized networks MN1 to MN5 are networks obtained in advance by training specialized for different information about input data, for example, the name, type, attribute, and related numerical values of an object. Fig. 5 shows examples of the types of nodules corresponding to the modularized networks MN1 to MN5, respectively, and of training images with respect to the types of the nodules trained for network construction, respectively. In these examples, a plurality of data sets (five sets in this example) including a plurality of images for each type of lung nodule appearing in CT images of a chest were prepared as training image data (also referred to as first training image data) for constructing the modularized networks MN1 to MN5. The five data sets included in the first training image data are trained by inputting into pretraining modularized networks MN1 to MN5, respectively, whereby trained modularized networks MN1 to MN5 are constructed. Thus, the modularized networks MN1 to MN5 are constructed as networks obtained by training of images of a small nodule, a large nodule, a roundish nodule, a nodule with a spiculated boundary, and a nodule with uniform internal density, respectively.

When the input data IN for the estimation object is input into the modularized networks MN1 to MN5 constructed as described above, the modularized networks MN1 to MN5 outputs output signals S1 to S5 indicating the extent to which the images of the nodules included in the input data IN correspond to the types of trained nodules, respectively, as estimation results, for example (step S12 in Fig. 3). The output signals S1 to S5 are, for example, values (that is, probabilities) indicating whether the nodules included in the input data IN match the types of nodules trained by the modularized networks, respectively, and may be output as values from 0 or more and 1 or less.

Next, the fusion network FN will be described. The fusion network FN is constructed in advance in such a manner that a plurality of CT images for training (second training image data) are input into the modularized networks MN1 to MN5, which are constructed in advance, and are subjected to supervised training. In other words, the fusion network FN is constructed in such a manner that a multidimensional vector having the output signals S1 to S5, which are the estimation results of the modularized networks MN1 to MN5, as components is input as an explanatory variable into the fusion network FN, and the type of a nodule to be output is given as an object variable to perform training.

Thereby, the fusion network FN estimates, based on the output signals S1 to S5 indicating the estimation results of the modularized networks MN1 to MN5, what types of nodules are included in the input data IN, and outputs estimation results (step S13 in Fig. 3).

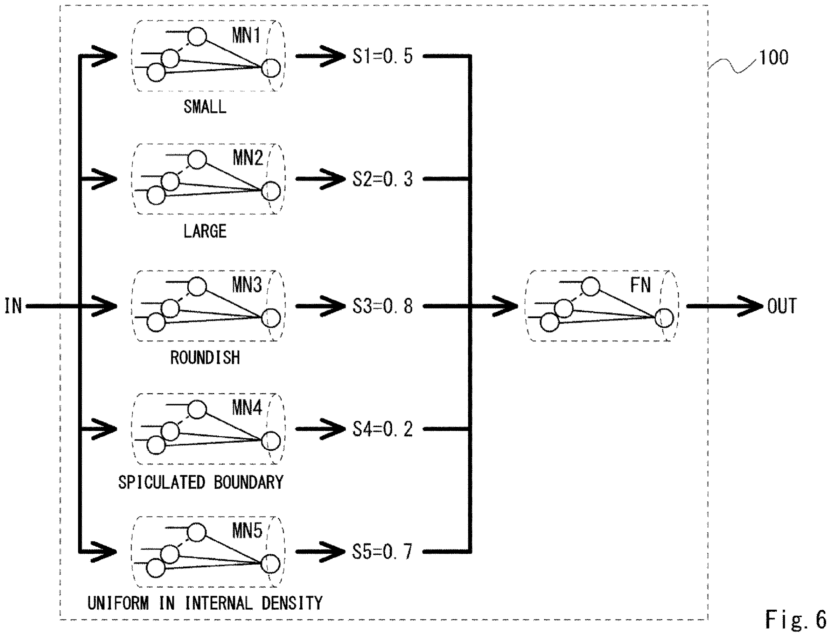

Fig. 6 schematically shows an example of estimation in the estimation apparatus 10. In this example, when the images of the chest CT are input, the values of the output signals S1 to S5 output by the modularized networks MN1 to MN5 are 0.5, 0.3, 0.8, 0.2, and 0.7, respectively. The multidimensional vector having the output signals S1 to S5 as components is input to the fusion network FN. In the fusion network FN constructed as a result of training, a boundary surface is formed in a vector space, to which the multidimensional vector belongs, to discriminate whether the nodule is "benign" or "malignant", and it is possible to determine whether the input multidimensional vector belongs to a "benign" region or a "malignant" region.

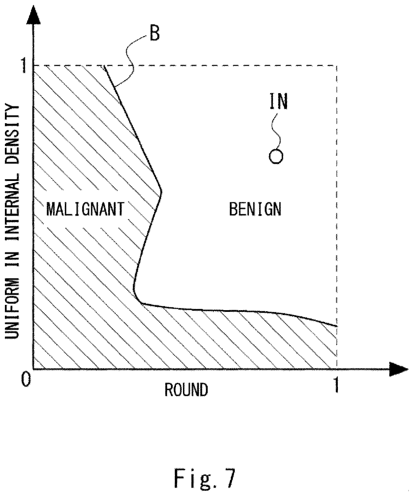

For example, in this example, the estimation results are output in which the type of nodule is "benign" and the reason for discrimination is that "the nodule is roundish and uniform in density". Fig. 7 shows an image of an estimation process in the example of Fig. 6. For the sake of simplicity, Fig. 7 focuses on and shows that the type of nodule is "roundish " (output signal S3) and "uniform in density" (output signal S5). As shown in Fig. 7, in this example, a two-dimensional plane is configured with an axis of the degree to which the nodule is "roundish " (the value of the output signal S3) and an axis of the degree to which the nodule is "uniform in density" (the value of the output signal S5), and a boundary line B is formed within the plane by training as the fusion network to demarcate whether the nodule of the estimation object indicated by the input data IN is "benign" or "malignant". In this example, since the input data IN exists in the "benign" region, the estimation result indicating that the nodule as the estimation object is "benign" is output.

Further, the reason for discrimination is created by reference of the values of the output signals S1 to S5. In this example, with reference to the two output signal values with high-level values, the reason for discrimination is constituted based on predominance of a characteristic of a lesion site which is "roundish " corresponding to the output signal S3 with the highest value (0.8) and of a characteristic of a lesion site which is "uniform in internal density" corresponding to the output signal S5 with the second highest value (0.7). The reason for discrimination obtained in this way is displayed on the image display unit 13. Needless to say, at the time of constitution of the reason for discrimination, the reason for discrimination may be constituted based only on the highest output signal, or may be constituted based on three or more output signals.

The boundary line B formed by the training of the fusion network in Fig. 7 indicates a place where a probability of certainty of benign and malignant as determined by the fusion network is exactly 50%, but the reason for discrimination can be constituted by calculation of the degree to which the output signals S1 to S5 contribute to the determination of benign (or malignant). The degree of contribution to the determination is calculated by multiplying the output signals S1 to S5 by weights of the networks combined to each other. When a sigmoid function is used in the fusion network, piecewise linear approximation is performed using a derived function thereof to calculate the weight for each of the output signals S1 to S5 of all the networks, and to create the degree of contribution of each of the output signals to the determination. Reason and basis may be constituted based on the degree of contribution obtained in this way.

In the present embodiment, the "reason" refers to information indicating why such a discrimination result was reached, and the "basis" refers to evidence such as an image used to constitute the reason. In the present embodiment, depending on the discrimination result, not only the reason but also the basis together with the reason may be displayed. Thus, a user can refer to the basis for the displayed reason to evaluate the validity of the reason.

Next, advantages of the estimation processing in the estimation apparatus 10 will be described. In a general trained model constructed by deep learning depending on an end-to-end machine learning paradigm, the name, type, attribute, and related numerical values of the lesion site can be estimated by inputting the input data into the trained model obtained by supervised training of the CT images and MRI images. However, in the general trained model, only the type of nodule in an image of the input data can be estimated, and it is unclear why the estimation result is obtained. For this reason, the user can only know the type of the nodule as an estimation result, and cannot obtain information for determining whether the estimated type of nodule is appropriate. This makes it difficult for users such as doctors who use the estimation result to make a diagnosis with confidence in the estimation result.

In contrast, the estimation apparatus according to the present embodiment inputs the input CT or MRI images to the modularized networks MN1 to MN5, estimates whether the nodule as the estimation object matches the characteristics of the nodule trained in advance, and estimates the type of nodule by further inputting the estimation result into the fusion network. Therefore, as in the example above, the nodule can be estimated to be "benign" based on the reason that the lesion site is "roundish " and "uniform in internal density", and the user can recognize the process and reason for the estimation. This can allow the user to compare the estimation reason and the estimated type of nodule and examine whether the estimation result is appropriate. As a result, the user can evaluate the estimation result from a professional perspective, and can determine whether the estimation result is reliable.

This has been made possible by separating and evaluating the characteristics of the nodule as the estimation object using the modularized networks MN1 to MN5 that have trained nodules with different characteristics. Therefore, it can be understood that the characteristics of the estimation object cannot be separated and evaluated using a method such as general deep learning or the like as in the present embodiment.

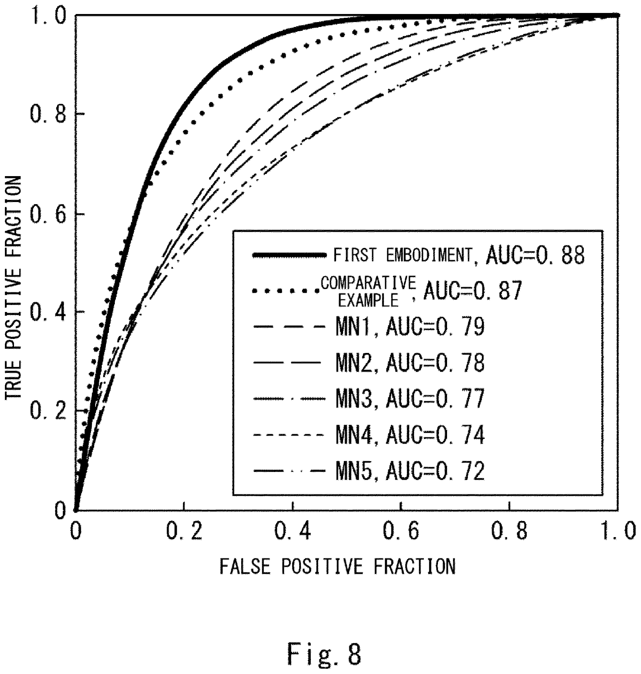

Next, examination will be made with respect to estimation accuracy in estimation processing using the estimation apparatus 10 according to the present embodiment and estimation accuracy in estimation processing using general deep learning. Note that, Fig. 8 shows estimation accuracy in estimation processing using the estimation apparatus according to the present embodiment and a general model. In Fig. 8, the estimation accuracy is displayed in AUC (Area Under Curve). As shown in Fig. 8, the AUC in a general estimation method (hereinafter, referred to as a comparative example) is 0.87, whereas the AUCs in estimation using only the modularized networks MN1 to MN5 are 0.7 range, which is inferior to the comparative example. On the other hand, when estimation is performed using the estimation apparatus 10, the AUC was 0.88, and it is clear that the estimation accuracy equivalent to that of the comparative example (AUC = 0.87) can be achieved.

As described above, according to such a configuration, it is possible to provide an estimation apparatus that can also output the reason for estimation while ensuring estimation accuracy equivalent to that of the estimation processing to which general deep learning is applied.

Further, the modularized network constructed in the present embodiment can be realized with a relatively small-scale network compared to a general deep learning model. In the general deep learning, it is also conceivable that there is a redundant network that is small in contribution to the estimation processing. In contrast, according to the present configuration, it is possible to configure the networks, which are small in scale and low in redundancy, by introduction of the modularized networks, and to easily know which modularized network is used for estimation.

### Second Embodiment

The case has been described in the first embodiment in which the estimation processing unit 12 receives the information indicating the trained model from the external memory apparatus or the like and holds the trained model based on the received information indicating the trained model. In contrast, a case will be described in the present embodiment in which the estimation processing unit 12 learns a model using data for training and constructs a trained model.

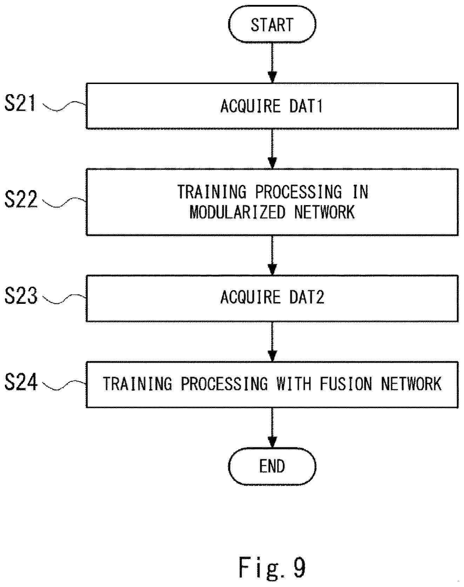

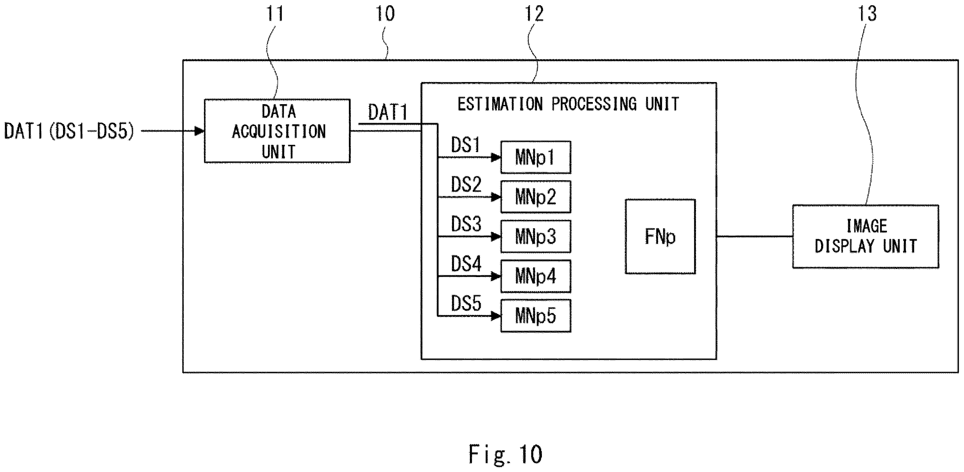

Fig. 9 shows a flowchart of training processing in the estimation apparatus 10 according to a second embodiment. Further, Fig. 10 schematically shows a first example of input/output of data in the estimation apparatus 10 in the second embodiment. In order to construct the trained model 100, the data acquisition unit 11 acquires first training image data DAT1 to construct modularized networks (step S21 in Fig. 9). The data acquisition unit 11 can appropriately read the first training image data DAT1 from a memory apparatus, a CT apparatus, and an MRI apparatus.

The read first training image data DAT1 is input to the estimation processing unit 12. The estimation processing unit 12 executes training by inputting data sets DS1 to DS5 included in the first training image data DAT1 and classified by the name, type, and attribute of an object into untrained modularized networks MNp1 to MNp5, respectively (step S22 in Fig. 9). Thereby, trained modularized networks MN1 to MN5 can be constructed.

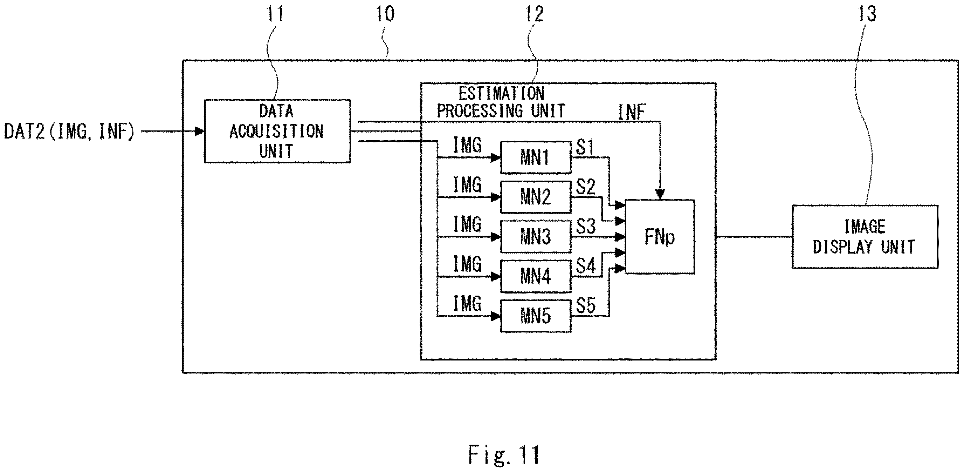

Then, the data acquisition unit 11 acquires second training image data DAT2 to construct a fusion network (step S23 in Fig. 9). Fig. 11 schematically shows a second example of input/output of data in the estimation apparatus 10 in the second embodiment. The data acquisition unit 11 can appropriately read the second training image data DAT2 from a memory apparatus, a CT apparatus.

The read second training image data DAT2 is input to the estimation processing unit 12. The image data DAT2 includes image data IMG of a plurality of chest CTs and type information INF indicating the type of nodule in each image included in the image data IMG. The estimation processing unit 12 inputs the image data IMG into the constructed and trained modularized networks MN1 to MN5. Thereby, output signals S1 to S5 are output to untrained fusion network FNp from the modularized networks MN1 to MN5, respectively. Thus, supervised training is performed using, as input data, the output signals S1 to S5 for each input image and information indicating the type of nodule in each image included in the type information INF (step S24 in Fig. 9). Thereby, a trained fusion network FN can be constructed.

As described above, according to the present configuration, the estimation processing unit 12 can also construct the trained model 100. Thereby, the construction of the trained model and subsequent estimation processing can be performed by a single estimation apparatus.

### Other Embodiments

Note that, the present invention is not limited to the above-described embodiments, and can be modified as appropriate without departing from the gist of the present invention. For example, the case has been described above in which the number of modularized networks is five, but this is merely an example, and two or more and four or less modularized networks, or six or more modularized networks may be used as appropriate.

The estimation related to the CT images has been described in the above-described embodiments, but images to be estimated are not limited to the CT images, and may be other medical images, for example, MRI images, X-ray images, ultrasound images, and nuclear medicine images. Further, the present invention is applicable not only to estimation of medical images but also to estimation of images in other fields.

In the above-described embodiments, the present invention has been mainly described as a hardware configuration, and however, it is not limited to this. Any processing can be achieved by causing a CPU (central processing unit) to execute a computer program. In this case, a computer program can be stored and provided to a computer by use of various types of non-transitory computer-readable media. Such non-transitory computer-readable media include various types of tangible storage media. Examples of such non-transitory computer-readable media include a magnetic storage medium (e.g., a flexible disk, a magnetic tape, a hard-disk drive), a magneto-optical recording medium (e.g., a magneto-optical disk), a CD-ROM (read-only memory), a CD-R, a CD-R/W, and a semiconductor memory (e.g., e a mask ROM, a programmable ROM (PROM), an erasable PROM (EPROM), a flash ROM, or a random-access memory (RAM)). Furthermore, a program may also be provided to a computer by use of various types of transitory computer-readable media. Examples of such transitory computer-readable media include an electric signal, an optical signal, and an electromagnetic wave. A transitory computer-readable medium can provide a program to a computer via a wired communication line, such as an electric wire or an optical fiber, or via a wireless communication line.

This application claims priority based on Japanese Patent Application No. 2021-195421 filed on December 1, 2021 and the disclosure of which is incorporated herein in its entirety.

### Reference Signs List

- DAT1: FIRST TRAINING IMAGE DATA
- DAT2: SECOND TRAINING IMAGE DATA
- DS1 TO DS5: DATA SET
- FN, FNp: FUSION NETWORK
- IN: INPUT DATA
- MN1 TO MN5, MNp1 TO MNp5: MODULARIZED NETWORK
- OUT: ESTIMATION RESULT
- S1 TO S5: OUTPUT SIGNAL
- 10: ESTIMATION APPARATUS
- 11: DATA ACQUISITION UNIT
- 12: ESTIMATION PROCESSING UNIT
- 13: IMAGE DISPLAY UNIT
- 100: TRAINED MODEL
- 1000: COMPUTER
- 1001: CPU
- 1002: ROM
- 1003: RAM
- 1004: BUS
- 1005: INPUT/OUTPUT INTERFACE
- 1006: INPUT UNIT
- 1007: OUTPUT UNIT
- 1008: MEMORY UNIT
- 1009: COMMUNICATION UNIT
- 1010: DRIVE
- 1011: MAGNETIC DISK
- 1012: OPTICAL DISK
- 1013: FLEXIBLE DISK
- 1014: SEMICONDUCTOR MEMORY

## Claims

1. An estimation apparatus comprising:
a data acquisition unit configured to acquire input data serving as image data;
an estimation processing unit configured to estimate information relating to an estimation object represented by a signal included in the input data by inputting the input data into a trained model; and
a display unit configured to display the estimated information relating to the estimation object and information indicating a reason why the estimated information relating to the estimation object is estimated,
wherein the trained model includes
a plurality of modularized networks constructed in such a manner that characteristics of the estimation object represented by a signal included in first training image data are divided by type and training is implemented in advance, and
a fusion network constructed in such a manner that a plurality of output signals obtained by input of second training image data into the plurality of modularized networks are input and supervised training is implemented, whereby estimating information relating to the estimation object represented by a signal included in an input image serving as image data, according to a signal to be input.

2. The estimation apparatus according to claim 1, wherein the information relating to the estimation object includes some or all of name, type, and attribute of the object and a numerical value related to the object.

3. The estimation apparatus according to claim 1 or 2, wherein the fusion network is constructed by training of a multidimensional vector having the plurality of output signals as components.

4. The estimation apparatus according to any one of claims 1 to 3, wherein the estimation processing unit further outputs information indicating the degree of influence of the plurality of output signals on a result of estimation.

5. The estimation apparatus according to claim 4, wherein the estimation processing unit selects the predetermined number of the output signals in descending order of the degree of influence on the result of estimation, and further outputs information indicating the selected output signals.

6. The estimation apparatus according to claim 5, wherein the display unit displays the information indicating the selected output signals as information indicating the reason or information indicating the reason and a basis on which the reason is obtained.

7. The estimation apparatus according to any one of claims 1 to 6, wherein the estimation processing unit is configured to
construct the plurality of trained modularized networks by receiving the first training image data from the data acquisition unit and inputting the first training image data into the plurality of modularized networks, and
construct the trained fusion network by receiving the second training image data from the data acquisition unit and inputting the plurality of output signals obtained by the input of the second training image data into the plurality of modularized networks into the fusion network to implement supervised training.

8. The estimation apparatus according to any one of claims 1 to 7, wherein
the first training image data includes a plurality of data sets trained in each of the plurality of modularized networks, the plurality of data sets corresponding to the information relating to the estimation object, and
the plurality of modularized networks, which have been trained, are constructed in such a manner that the plurality of data sets are inputted into the plurality of modularized networks to be subjected to training, respectively.

9. The estimation apparatus according to any one of claims 1 to 8, wherein each of the plurality of output signals obtained by inputting the second training image data into the plurality of modularized networks is a signal corresponding to one type of the characteristics of the estimation object.

10. The estimation apparatus according to any one of claims 1 to 9, wherein the estimation processing unit estimates, as the information relating to the estimation object, discrimination information relating to a state of the estimation object discriminated based on a type of the characteristics of the estimation object.

11. The estimation apparatus according to claim 10, wherein the fusion network constructed by training of the first training image data and the second training image data includes a boundary surface that is formed in a vector space, to which a multidimensional vector having the output signals from the plurality of modularized networks belongs, to discriminate the state of the estimation object.

12. An estimation method comprising:
acquiring input data serving as image data;
estimating information relating to an estimation object represented by a signal included in the input data by inputting the input data into a trained model; and
displaying the estimated information relating to the estimation object and information indicating a reason why the estimated information relating to the estimation object is estimated,
wherein the trained model includes
a plurality of modularized networks constructed in such a manner that characteristics of the estimation object represented by a signal included in first training image data are divided by type and training is implemented in advance, and
a fusion network constructed in such a manner that a plurality of output signals obtained by input of second training image data into the plurality of modularized networks are input and supervised training is implemented, whereby estimating information relating to the estimation object represented by a signal included in an input image serving as image data, according to a signal to be input.

13. A program causing a computer to execute processes of:
acquiring input data serving as image data;
estimating information relating to an estimation object represented by a signal included in the input data by inputting the input data into a trained model; and
displaying the estimated information relating to the estimation object and information indicating a reason why the estimated information relating to the estimation object is estimated,
wherein the trained model includes
a plurality of modularized networks constructed in such a manner that characteristics of the estimation object represented by a signal included in first training image data are divided by type and training is implemented in advance, and
a fusion network constructed in such a manner that a plurality of output signals obtained by input of second training image data into the plurality of modularized networks are input and supervised training is implemented, whereby estimating information relating to the estimation object represented by a signal included in an input image serving as image data, according to a signal to be input.
